# EUROPÄISCHE PATENTSCHRIFT

(11) **EP 0 615 618 B1**
(45) Veröffentlichungstag und Bekanntmachung des Hinweises auf die Patenterteilung: **15.04.1998**
(21) Anmeldenummer: 93900029.5
(22) Anmeldetag: 07.12.1992
(51) Int. Cl.: G01N 21/64, G01N 21/76, G01N 33/18

(54) **VERFAHREN UND VORRICHTUNG ZUR MESSUNG DER FLUORESZENZRÜCKMELDUNG VON ALGEN**
PROCESS AND DEVICE FOR MEASURING ALGAE FLUORESCENCE FEEDBACK
PROCEDE ET DISPOSITIF DE MESURE DE LA FLUORESCENCE EN RETOUR D'ALGUES

(30) Priorität: 07.12.1991 DE 4140414
(43) Veröffentlichungstag der Anmeldung: 21.09.1994
(73) Patentinhaber: MOLDAENKE, Christian, D-24103 Kiel (DE)
(72) Erfinder: MOLDAENKE, Christian, D-24103 Kiel (DE)
(74) Vertreter: Einsel, Robert, Dipl.-Ing.
(86) Internationale Anmeldenummer: EP9202828
(87) Internationale Veröffentlichungsnummer: WO9312415

(56) Entgegenhaltungen:
- EP-A- 0 209 247
- DE-A- 3 518 527
- DE-A- 3 715 114
- GB-A- 2 167 181
- US-A- 3 754 145
- PHOTOSYNTHESIS RESEARCH Bd. 10, 1986, Seiten 51 - 62 U. SCHREIBER ET AL. 'Continuous recording of photochemical and non-photochemical chlorophyll fluorescence quenching ...'

## Beschreibung

Die Erfindung betrifft ein Verfahren und eine Vorrichtung zur Messung der Fluoreszenzrückmeldung von Algen zur Algendichte- und Vitalitätsbestimmung mit einer anregenden Beleuchtung, bei dem die Fluoreszenzabstrahlung der untersuchten Pflanzen detektiert wird,

Es ist bereits ein Verfahren bekannt (DE 37 15 114 A1), bei dem die Fluoreszenzrückmeldung von Pflanzen gemessen wird. Mit Hilfe der Fluoreszenzrückmeldung kann man Aussagen über die Veränderung des Pflanzenzustandes durch Änderung der Lichtintensität, Einfluß von Giften, Austrocknung, Temperaturänderungen und andere Umwelteinflüsse treffen. Eine Verwirklichung dieser Verfahren sind blitzlichtinduzierte Messungen zur Fluoreszenzrückmeldung oder das aus der genannten DE 37 15 114 A1 bekannte Verfahren, bei dem eine Korrelation zwischen einer prompten Fluoreszenz und einer verzögerten Fluoreszenz bestimmt wird und dazu zwei separate Fluoreszenzmeßeinrichtungen mit jeweils einer eigenen Meßkammer verwendet werden.

Es ist auch ein Fluorimeter bekannt (DE 35 18 527 A1), bei dem eine periodisch gepulste Lichtquelle zur Fluoreszenzanregung von lebenden, einem Starklicht wie Sonnenlicht ausgesetzten Pflanzen genutzt wird und durch Messung der Fluoreszenzrückmeldung eine Aussage insbesondere über die Veränderung der Chlorophyllfluoreszenz getroffen wird.

Es ist Aufgabe der Erfindung, ein Verfahren und eine Vorrichtung zu entwickeln, innerhalb kurzer Zeit und einfach die Algendichte, d.h.die Abschwächung des Lichtes in einer Probe, und die Vitalität der Pflanzen relativ genau bestimmt werden kann. Dabei soll auf eine Messung der verzögerten Fluoreszenz ebenso wie auf separate Fluoreszenzmeßeinrichtungen völlig verzichtet werden.

Diese Aufgabe wird bei einem Verfahren der eingangs beschriebenen Art durch folgende Verfahrensschritte gelöst:
- Beleuchtung der Algen gleichzeitig mit zwei verschieden getakteten Lichtsignalen, einem schnell getakteten Meßlicht und einem zweiten langsam getakteten x-Hz-Licht,
- Korrelieren des detektierten Signals mit dem Taktsignal des Meßlichts zum Erhalt eines für die Algendichte maßgeblichen Zwischensignals und
- Korrelieren des Zwischensignals mit dem Taktsignal des x-Hz-Lichts zum Erhalt eines eichbaren, für die Vitalität maßgeblichen Ergebnissignals,
- Integrieren beider Signale.

Vorteilhafte Weiterbildungen des Verfahrens sind in den Unteransprüchen beschrieben.

Dieses Verfahren, bei dem gleichzeitig verschieden getaktete Lichtsignale aufgebracht werden, ermöglicht es, nach dem Korrelieren aus dem Signal, das die Algen abgeben, festzustellen, wie schnell die Algen auf das eingestrahlte Licht reagieren bzw. daraus wiederum, wie viele Algen in einem bestimmten Volumen vorhanden sind. Auch läßt sich die Algenvitalität bestimmen. Durch die doppelte Korrelation ist es nicht unbedingt nötig, Filter vor Lichtquellen und Detektor zu verwenden, um das eingestrahlte Licht nicht gleich wieder zu detektieren. Mit dieser Methode wird es "herauskorreliert".

Außerdem kann auf eine sonst übliche und notwendige Linearisierung verzichtet werden, was sich insbesondere in der Meßzeit auswirkt. Mit dem erfindungsgemäßen Verfahren konnten somit Meßergebnisse erzielt werden, die um ein bis drei Zehnerpotenzen besser als die bisher bekannten Meßverfahren sind. Die Ergebnisse konnten während drei bis zehn minutenlanger Meßzeiten erzielt werden und liegen somit deutlich unter den Meßzeiten bisher bekannter Verfahren. Neben der großen Effektivität und schnellen Einsatzbereitschaft sind ist es besonders vorteilhaft, daß extrem gute Messungen auch schon mit Meßvolumina von weniger als 10 - 20 ml erzielt werden können. Bei einem kleinem Meßvolumen kann auch eine räumlich kleine und leichte Meßapparatur ermöglicht werden.

Werden die Algendichte bestimmt und damit die sich dadurch einstellnenden Abschwächung, so lassen sich Abschwächungseffekte herausrechnen, was die Effektivität und Genauigkeit der Messung weiter erhöht.

Zwar sind bei der Signalform des Meßlichts und des x-Hz-Lichtes alle bekannten Signalformen möglich, besonders gut sind jedoch Rechtecksignalformen geeignet, die sich sehr einfach und exakt erzeugen lassen und somit die Meßapparatur vereinfachen. Rechtecksignale lassen sich z.B. mittels eines einfachen Mikroprozessors sehr genau herstellen.

Neben der Algenvitalität können mit dem erfindungsgemäßen Verfahren außerdem Gehalte von Nitrat, Ammonium, Schwermetalle, Phosphor und weiterer Umweltgifte oder Stoffe in einer Flüssigkeit wie mit Algen durchsetztem Wasser ermittelt und somit mittels nur einer Messung eine umfassende und exakte Aussage über die Belastung eines natürlichen Gewässers oder Abwassers, z.B.aus einer Kläranlage, erhalten werden.

Weiter wird eine Vorrichtung zur Durchführung des Verfahrens vorgeschlagen, die sich dadurch auszeichnet, daß die zwei anregenden Lichtsignale aus unterschiedlichen Lichtquellen stammen. Durch die Verwendung zweier unterschiedlicher Lichtquellen, die jedoch nicht zwingend Voraussetzung ist, ist das Abgeben unterschiedlich getakteter Signale erheblich erleichtert. Andernfalls müßte eine einzelne verwendete Lichtquelle sich völlig linear verhalten, was technisch nur mit einem Regelsystem erreichbar ist.

In einer bevorzugten Ausführung besitzen die anregenden Lichtsignale eine Wellenlänge von weniger als 700 nm. Eine derartige Wellenlänge hat sich bei Algen als optimal erwiesen.

Eine besonders bevorzugte Ausführung zeichnet sich dadurch aus, daß Leuchtdioden die anregenden Lichtsignale erzeugen und diese eine Wellenlänge von 660 nm besitzen.

Eine derartige satte Färbung des eingestrahlten Lichts ist der beste Kompromiß zwischen markterhältlichen Leuchtdioden und maximaler Aufnahmefähigkeit durch die Algen.

Weiter wird vorgeschlagen, daß das erste, das Meßlichtsignal, mehrere Zehnerpotenzen schneller als das zweite Lichtsignal getaktet ist. Durch die Vorsehung dieser Differenz von mehreren Zehnerpotenzen zwischen den Taktraten der beiden signale wird die Auswertung erleichtert.

Hierbei wird insbesondere vorgeschlagen, daß das erste, das Meßlichtsignal, im Bereich von 1 kHz getaktet ist, und das zweite, das x-Hz-signal mit einer Frequenz im Bereich von 0,2 bis 5 Hz getaktet ist.

Zur Korrelation mit den Taktsignalen der Beleuchtungen werden jeweils phasenrichtige Gleichrichter vorgeschlagen. Eine derartige Schaltung ist sehr einfach aufzubauen und erfüllt den Zweck optimal.

Weiter wird für die Befüllung einer Meßkammer mit einer Zuflußöffnung und einer Abflußöffnung vorgeschlagen, eine Fördereinrichtung an der Abflußöffnung vorzusehen. Eine Vorsehung dort eignet sich für die Befüllung insbesondere deshalb, weil die in die Meßkammer eintretenden Algen so keiner mechanischen Belastung ausgesetzt werden. Dies hatte sich bisher als Problem herausgestellt, denn immer dann, wenn die Algen auf dem Weg zu der Meßkammer durch eine Pumpe hindurchtreten mußten, wurde ein nicht unbedeutender Teil von ihnen beschädigt.

Bezüglich des Aufbaus der Meßkammer wird weiter vorgeschlagen, daß die Meßkammer mit einer Mehrzahl von sich gegenüberliegenden, in direktem Kontakt zum Meßvolumen stehenden, Leuchtdioden versehen ist.

Wenn sich die Meßsignallichtquelle und die x-Hz-Lichtquelle gegenüberliegen, wird ein möglichst großes Volumen der Algen in der Meßkammer von beiden Lichtquellen durchsetzt, so daß ein sehr großes Durchsetzungs- bzw. Lichtmischvolumen entsteht, in dem die Algen mit dem Meßlicht und gleichzeitig dem x-Hz-Licht anregt werden. Durch das große Durchsetzungsvolumen wird eine große Menge Algen mit dem Licht beider Lichtquellen angeregt, was die Meßzeit erheblich verkürzt. Bei Anordnungen, bei denen beide Lichtquellen von der gleichen Seite die Algen bestrahlen, ist das mögliche Durchsetzungsvolumen erheblich kleiner, so daß schon allein deshalb eine Messung erheblich länger dauern muß, um brauchbare Ergebnisse zu erzielen.

Leuchtdioden haben in Hinblick auf die Erwärmung der Lösung den großen Vorteil, daß sie wenig Wärme abgeben und sehr kleine räumliche Ausmessungen haben, dabei einen extrem günstigen Wirkungsgrad haben, also evtl. sie mit Spannung versorgende Batterien nur sehr gering entleeren und daß sie zum Betrieb nur niedrige elektrische Spannungen benötigen. Gerade in Hinblick auf die Verwendung in der Nähe von Wasser, ist ein Verzicht auf hohe Spannungen sehr vorteilhaft.

Durch die Vorsehung von sich gegenüberliegenden Leuchtdioden wird es möglich, die Algen bestmöglich mit einer geringen Anzahl von Leuchtdioden auszuleuchten. Dabei fällt nicht direkt Licht auf die Detektoreinheit.

Es ist auch vorteilhaft eine Messung mit mehreren verschiedenen Leuchtdioden, die z.B. ein blaues, rotes, oranges, gelbes, grünes usw. Licht erzeugen. Wird bei einer Messung nacheinander oder zugleich mit entsprechend geigneten Lichtquellenpaaren z.B. rot - rot, blau - blau usw. angeregt, kann aus dem Ergebnis einer Meßreihe nicht nur eine Aussage über die Algenvitalität und Toxizitität getroffen werden, sondern sogar die Algenart und deren Quantität aus dem Vergleich der Meßergebnisse mit bekannten Referenzwerten bestimmt werden.

Zur Verhinderung von Übersteuerung werden optische Filter vor den Leuchtdioden mit einer oberen Grenzwellenlänge vorgeschlagen, unterhalb der sie Lichtwellen durchlassen. Durch derartige Filter ist es möglich, in einfacher konstruktiver Weise völlig sicherzustellen, daß eine Übersteuerung der Detektoreinheit stattfindet. Sie dienen also nur dem Schutz des Detektors und sind nicht zwingend nötig.

Bezüglich der Anordnung der Detektoreinheit wird vorgeschlagen, eine im rechten Winkel zu der Anordnung der Leuchtdioden vorgesehene Photodiode zur Messung des abgestrahlten Fluoreszenzlichts der Algen vorzusehen. Diese Geometrie ist konstruktiv einfach und leicht zu realisieren. Weiter wird vor der Photodiode ebenfalls in einer vorteilhaften Ausführung ein optischer Filter mit einer unteren Grenzwellenlänge vorgesehen, oberhalb der der Filter durchlässig ist. Mit einem solchen Filter wird, insbesondere im Zusammenwirken mit den anderen Filtern vor den Leuchtdioden sichergestellt, daß kein direktes Meßlicht in die Photodiode einfällt und gemessen wird.

Insbesondere wird vorgeschlagen, daß die Grenzwellenlängen der verschiedenen Filter gleich sind und bei 705 nm liegen.

Damit ist ein Wert geringfügig oberhalb des Grenzwertes für die fluoreszenzanregende Strahlung gelegt worden, jedoch noch am Rande des Bereichs in dem die Algen abstrahlen. Dieser ist nämlich von 700 bis 750 nm gemessen.

Ergänzend wird vorgeschlagen, mit einem Sensor die Stärke des photosynthetisch nutzbaren Hintergrundlichts zu messen. Falls die Meßkammer in situ, d.h. zum Beispiel unter Wasser, Anwendung findet , wäre die Anordnung des Sensors beliebig im Bereich der Meßkammer. Falls jedoch die Meßkammer außerhalb des Wassers betrieben wird, wäre die Anbringung dieses Sensors im Bereich des Ortes, an dem die Proben entnommen werden, zweckmäßig. Die von den Algen aufgenommene Lichtmenge in einem Zeitbereich von 2 - 3 Minuten vor der Messung beeinflußt das Floureszenzabgabeverhalten mit, wenn auch nur in vergleichsweise geringer Stärke.

Es wird auch vorgeschlagen, einen Temperatursensor an der Meßkammer vorzusehen, um beim Eichen der Meßkammer auch die Temperatur mitzumessen und so ein aktinisches Signal zu erhalten, daß sich auf den Chlorophyllgehalt eichen läßt.

Zur Vermeidung von Fremdlichteinfall auf das Flüssigkeitsvolumen mit den Algen sollte die Meßkammer gegen einen solchen abgeschlossen sein.

Besonders vorteilhaft ist es auch, die Meßkammer innenseits so auszubilden, daß sich keine Teile unerwünscht am Kammergrund ablagern und somit eine Messung verfälschen. Es daher vorteilhaft am Boden der Meßkammer einen Ablaß für schwebende und absinkende Teile vorzusehen und an der Oberseite der Meßkammer einen Auslaß für Gasteile wie z.B. Luft vorzusehen. Mit dieser Maßnahme wird die Genauigkeit einer Messung erhöht und der störende Einfluß von Fremdpartikeln, wie z.B. Sand und Luft, in einer Meßprobe deutlich verringert.

Mit der erfindungsgemäßen Vorrichtung läßt sich eine kompakte Meßstation aufbauen, die praktisch überall einsatzbereit ist. Wegen des geringen Energieverbrauchs der Meßstation ist ihr Betrieb über einen längeren Zeitraum automatisch und ohne längere Kontrolle möglich. Dazu ist in der Meßstation ein Mikroprozessor oder Rechner vorgesehen, der die Takt- und Meßsignale erzeugt und alle anderen Einrichtungen wie z.B. Ventile, Pumpen,Spül- und Reinigungseinrichtungen usw. steuert. Außerdem hat der Mikroprozessor oder Rechner die Funktion einer Datenerfassungs- und verarbeitungsstelle, in der alle gemessenen Daten des Photodetektors, der Dichte- und Abschwächemessung, sowie Meßeinrichtungen zur Ermittlung des Drucks, der Leitfähigkeit, des Sauerstoffs, der Temperatur, des Hintergrundlichtes usw. zu erfassen und zu verarbeiten und/oder in einem Speicher z.B. vom Typ SRAM abzuspeichern. Zur Energieversorgung des Meßstation und aller ihrer Einrichtungen ist bereits ein einfaches photovoltaische Element oder ein Akkumulator ausreichend. Es ist auch möglich, die Meßstation auf einem Ponton anzuordnen, so daß auch auf See Dauerrnessungen möglich sind. Zur Auswertung und Aufbereitung der Meßergebnisse kann der Mikroprozessor oder Rechner mit einem leistungsfähigen Computer verbunden werden. Ebenso ist es mittels einer Sendeeinrichtung in der Meßstation möglich, die gemessenen Daten zu einem bestimmten Empfänger zu schicken, der mit einer Auswerteeinrichtung verbunden ist.

Abschließend wird vorgeschlagen, die Meßkammer zur Aufnahme einer Küvette zwischen den Leucht- und Photodioden einzurichten. Damit wäre sie auch einfach im Labor nutzbar.

Weitere Merkmale und Vorteile der Erfindung ergeben sich aus der nachfolgenden Beschreibung eines bevorzugten Ausführungsbeispiels der Erfindung, das anhand der beiliegenden Zeichnung näher erläutert wird. Dabei zeigt:
- Fig. 1: den schematischen Aufbau der Vorrichtung, und
- Fig. 2: eine perspektivische Skizze der Meßkammer.
- Fig. 2b: perpektive Darstellung der Meßkammer mit dem herausgestellten Durchdringungsvolumen (gestrichelte Säule)
- Fig. 3: Ein Signaldiagramm der Korrelation bei aktivem und inaktiven Photosystemen
- Fig. 4: ein prinzipieler Aufbau einer automatischen Meßstation
- Fig. 5: ein Blockschhaltbild einer Meßstation
- Fig. 6: ein Querschnitt durch die Meßkammer
- Fig. 7: eine perspektivische Darstellung der Meßkammer mit einer Atenuationsmeßeinrichtung

Das Verfahren zur Messung der Fluoreszenzrückmeldung von Algen zur Dichte- und Vitalitätsbestimmung mit einer anregenden Beleuchtung besteht im wesentlichen aus den vier Verfahrensschritten : Beleuchtung der Algen mit zwei verschieden getakteten Lichtsignalen einem schnell getakteten Meßlicht mit einem zweiten relativ langsamen getakteten x-Hz-Licht, Detektieren der Fluoreszenzabstrahlung der Algen, Korrelieren des detektierten Signals mit dem Taktsignal des Meßlichtes zum Erhalten eines Zwischensignals und Korrelieren des Zwischensignals mit dem Taktsignal des x-Hz-Lichtes zum Erhalten eines eichbaren Ergebnissignals.

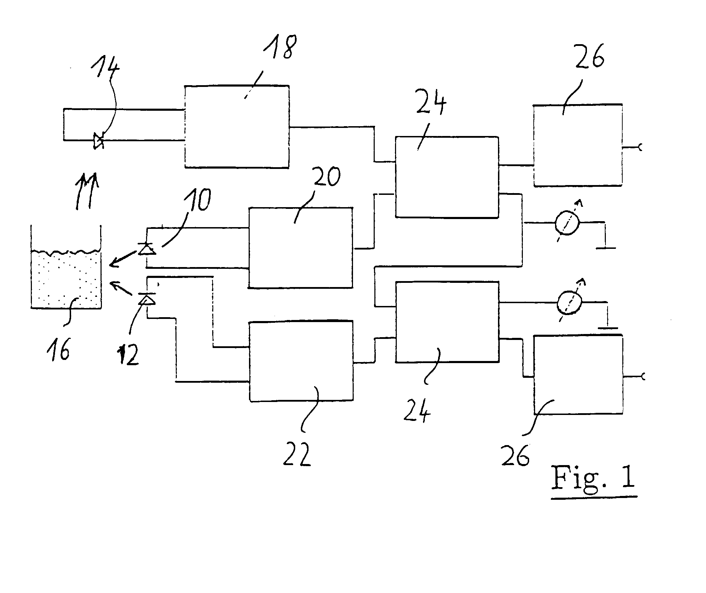

Dieses Verfahren beruht auf der bekannten Erkenntnis, daß chlorophyllhaltige lebende Materie variable Fluoreszenz abstrahlen, sobald die momentan eingestrahlte Energie nicht vollständig photosynthetisch genutzt werden kann. Die Fluoreszenzstrahlung tritt als Strahlung im Wellenlängenbereich zwischen 700 nm und 750 nm auf, während photosynthetisch nutzbar und fluoreszenzanregend die Strahlung bis 700 nm Wellenlänge ist. Das Verfahren arbeitet nun mit zwei verschieden getakteten Beleuchtungsquellen 10, 12, die in Form von 660 nm-Leuchtdioden realisiert sind. Diese sind in Fig. 1 durch das Symbol für Dioden dargestellt. Die erste Leuchtdiode 10 wird mit einer Frequenz von 1 kHz angesteuert und als Meßlicht bezeichnet. Die zweite Leuchtdiode 12 wird mit einer Frequenz zwischen 0,2 und 5 Hz betrieben und als x-Hz-Licht bezeichnet. Die hinter den Leuchtdioden 10, 12 angeordneten Kästen 20, 22 im Prinzipschaltbild sind die Logik 20 zur Erzugung des 1 kHz-Signales bzw. die Logik 22 zur Erzeugung des x-Hz-Signales. Bezugszeichen 24 bezeichnet dann jeweils einen Verstärker und Korrelator, deren Ausgangssignale dann auf den nächsten Verstärker und Korrelator und einen Integrator 26 und eine Anzeigeeinrichtung geleitet werden.

Mit dem integrierten Signal nach der ersten Korrelation kann eine Normierung z. B. auf die im Wasser enthaltenen Gelbstoffe geschehen, die ebenfalls fluoreszieren können.

Die Meßlichtimpulse bewirken bei den im Meßvolumen befindlichen Algen 16 eine Fluoreszenzantwort, die über die Photozelle 14 aufgenommen wird, an den Detektor und Verstärker 18 weitergegeben wird und dann durch die obengenannten Mittel weiterverarbeitet, d. h. verstärkt und mit dem 1 kHz Taktsignal des Meßlichtes korreliert wird.

Die Stärke des auf einen Lichtreiz emittierten Signals ist bei chlorophyllhaltigem Material abhängig von verschiedenen Bedingungen, wie Hintergrundlicht, Temperatur, Giftgehalt, Algenart und Algendichte. Das zusätzliche x-Hz-Licht erweitert das Fluoreszenzmeßverfahren zu einem Verfahren zur Vitalitätsbestimmung. Änderungen der Gesamtintensität bewirken Änderungen der photosynthetischen Aktivität, die den Wirkungsgrad der Fluoreszenzabstrahlung und damit auch die Größe des gemessenen Signales beeinflussen. Damit dienen die Hintergrundlichtinduzierten Schwankungen des Meßlichtsignals als Maß für die Funktionsfähigkeit des photosynthetischen Apparates.

Diese Veränderungen des Hintergrundlichtes erzeugt die Leuchtdiodenanordnung des x-Hz-Lichtes. Die dadurch hervorgerufene Modulation des gemessenen Signals wird mit den phasenrichtigen Gleichrichtern (Korrelatoren) ermittelt und als aktinisches Signal bzw. Maß für die Vitalität ausgegeben.

Die Größe der Algenvitalität in einer Meßkammer definierten Volumens ist sehr stark abhängig von der enthaltenen Chlorophyllmenge bzw. der Algenzahl.

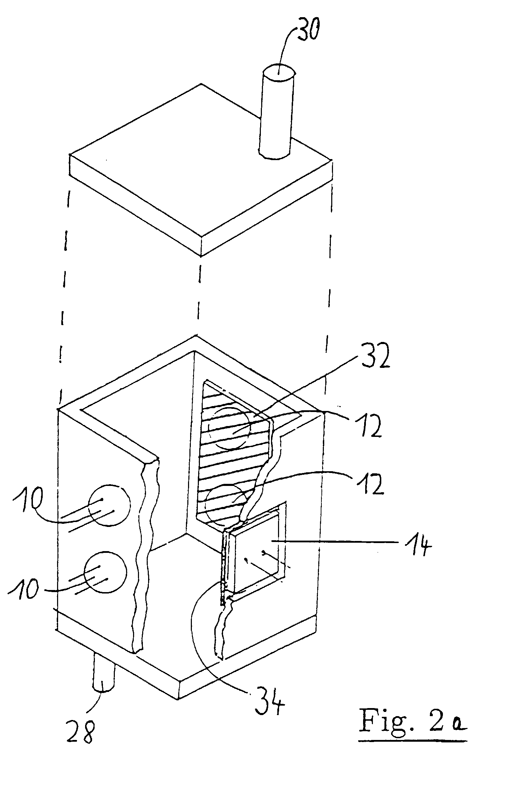

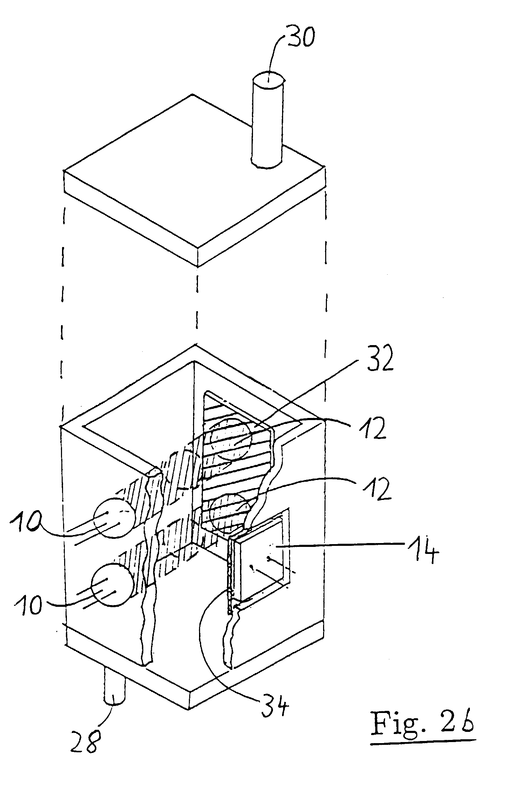

Das Betreiben der vorgeschlagenen Vorrichtung geschieht folgendermaßen: Eine Algenlösung, z. B. Meerwasser, wird in eine Kammer, wie sie beispielsweise in Fig. 2 dargestellt ist, gepumpt und für eine bestimmte Zeit im Minutenbereich den Beleuchtungen der Dioden 10, 12 ausgesetzt. Da in diesem Fall kein Hintergrundlicht, wie z. B. Sonnenlicht, in die Kammer fallen kann, herrschen in der Kammer sehr konstante definierte Lichtverhältnisse, das Ausgangssignal wird dadurch sehr störunempfindlich.

Die alleinige Nutzung von Leuchtdioden 10, 12 als Beleuchtungsquellen bietet den weiteren Vorteil, daß sie große Schwierigkeiten in Hinblick auf Erwärmung der Lösung während der Messung vermeidet. Leuchtdioden sind außerdem sehr klein und besitzen extrem günstigen Wirkungsgrad, wobei die benötigten elektrischen Spannungen sehr niedrig sind. Gerade im Feldeinsatz in der Nähe von Meerwasser wird es sich als sehr vorteilhaft erweisen, wenn geringe Spannungen und ein günstiger Wirkungsgrad der, Beleuchtung vorhanden sind, da dann kleinere Batterien verwendet werden können.

Die Beleuchtung der Algen für einen längeren Zeitraum vor der Messung spielt für deren Fluoreszenzantwort ebenfalls eine Rolle. Daher sollte diese Beleuchtung, der die Algen ausgesetzt waren, erfaßt werden. Weiter ist die Temperatur der Algen ein bestimmender Faktor für das Maß der Fluoreszenzrückmeldung. Sie wird daher auch zu messen sein, wodurch das gemessene aktinische Signal sich nun auf den Chlorophyllgehalt eichen läßt.

Die Kammer läßt sich über eine Zuflußöffnung 28 füllen, wobei die vorher in der Kammer befindliche Meßflüssigkeitsvolumen durch die Abflußöffnung 30 hinausgedrückt wird. Vorteilhaft wäre eine tangentiale Einströmung im unteren Bereich und eine tangentiale, der Drehrichtung der Flüssigkeit folgende Abführung im oberen Bereich.

Es werden in Abhängigkeit von der Anzahl und Größe der Leucht- und Photodioden für die Meßkammer Maße von etwa 40x40x40 mm vorgeschlagen. Natürlich kann dies für andere Anwendungszwecke erheblich variiert werden.

Die Leuchtdioden 10, 12 und die detektierende Photodiode 14 werden direkt, ohne schwächende Lichtleiter, sondern nur durch vor Übersteurung schützende optische Filter 32, 34 von dem Meßvolumen getrennt, direkt an das Meßvolumen anschließend vorgesehen. Für die Filter 32 vor den Leuchtdioden 10, 12 wird eine obere Grenzwellenlänge für durchgelassenen Lichtwellen von 705 nm vorgeschlagen, für den Filter 34 vor der Photodiode 14 eine untere Grenzwellenlänge von 705 nm. Je nach Bedarf können mehrere Leuchtdioden 10; 12 dasselbe Signal aussenden, wie es in Fig. 2 durch die vier anregenden Leuchtdioden 10, 10, 12, 12 angedeutet wird.

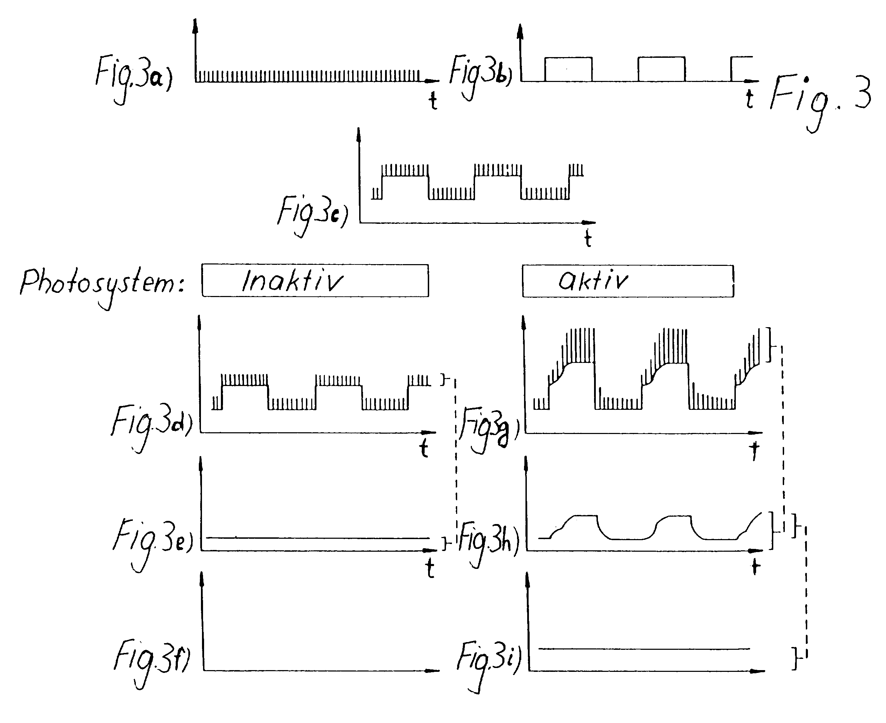

Fig 3 zeigt anschaulich das unterschiedliche Korrelationsverhalten aktiver Photosysteme wie z.B. Algen und inaktiverPhotosysteme. In Fig 3a und 3b sind Rechtecksignale des 1 kHz Meßlichts und eines 1 Hz Lichtes gezeigt, mit denen jeweils Leuchtdioden angeregt werden. Fig. 3 c zeigt die Addition beider Signale, wie sie in der Meßkammer erfolgt. Für die Anregung der Algen ist hauptsächlich das überlagerte Signale von Bedeutung.

Fig 3 d -f zeigt das Ergebnis eines inaktiven Photosystem mit der Fluoreszenzantwort in Fig. 3d, nach der ersten Korrelation , dem sogannten Fluoreszenz yield, in Fig. 3e und nach der zweiten Korrelation, dem sogenannten Aktivitätssignal, in Fig. 3f. Bei photoinaktiven Systemen ist das Ergebnis des Aktivitätssignals nach der zweiten Korrelation stets null. Somit werden alle Einflüsse, die durch inaktive Photosysteme erzeugt werden könnten, "herauskorreliert".

Fig. 3 g - i zeigt das Korrelationsverhalten aktiver Photosysteme. Nach der ersten Korrelation bleibt die Hüllkurve des in Fig. 3 gezeigten 1 kHz übrig. Nach der zweiten Korrelation, stellt sich ein Wert größer Null als Maß für die Aktivität und Vitalität der Algen ein. Alle Korrelationen der Doppelkorrelation sind vorzugsweise Kreuzkorrelationen. Mit Messungen von einer zeitlichen Länge von etwa fünf Minuten konnten Ergebnisse erzielt werden, die um mehrere Zehnerpotenzen genauer sind als bei bisher bekannten Verfahren, die zudem erheblich zeit- und vorrichtungsaufwendiger sind.

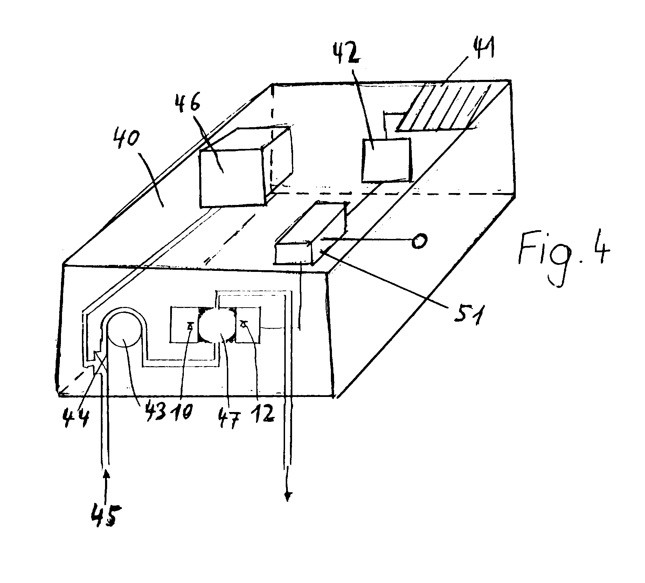

Fig 4 zeigt ein Prinzipbild einer in einem schwimmfähigen Ponton eingebauten automatischen Meßstation 40. Zur Energieversorgung der Meßstation, die im Schnitt weniger als 5 W Energie benötigt, und aller seiner Einrichtungen ist ein photovoltaisches Element 41 und ein daran angeschlossener Akkumulator 42 vorgesehen. Mittels einer Pumpe 43 wird je nach Ansteuerung eines Umschaltventils 44 entweder Probenwasser 45 oder Spül/Reinigungsflüssigkeit 46 in die Meßkammer 47 befördert. Nach einer Messung wird die Meßkammer mit der Spülflüssigkeit gereinigt und ist somit für den nächsten Meßvorgang bereit.

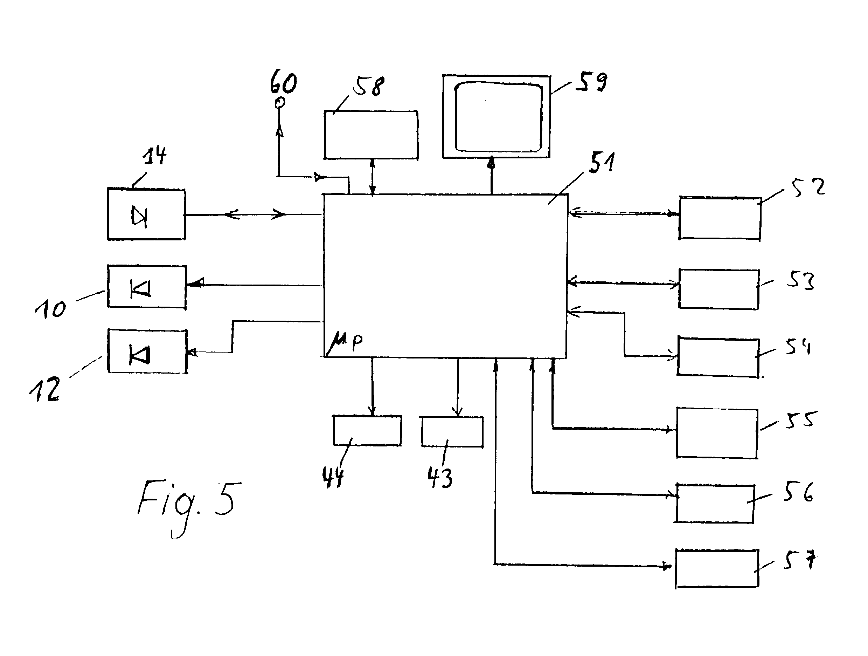

Fig. 5 zeigt ein Blockschaltbild der Dauermeßstation 40 mit einem Rechner oder Mikroprozessor 51 vom Typ SIEMENS 8051 zur Erfassung und Auswertung der gemessenen Daten des an der Meßkammer angeordneten Photodetektors 14, einer Dichte- oder Abschwächungs- oder Atenuationsmeßeinrichtung 52, einer Druckmeßeinrichtung 53, einer Leitfähigkeitsmeßeinrichtung 54, einer Sauerstoff- bzw. Gasmeßeinrichtung 55, einer Temperaturmeßeinrichtung 56, einer Hintergrundlichtmeßeinrichtung 57 sowie weiterer angeschlossener Meßeinrichtungen wie z.B. Uhrzeit. Alle gemessenen und/oder ausgewerteten Daten werden in einem als Speicher 58 vom Typ SRAM (Static Random Access Memory) ausgebildeten Datensammler gespeichert. Zur Anzeige der Daten ist eine Anzeigeeinrichtung 59 vorgesehen. Des weiteren weist die Meßstation eine Schnittstelle zu einer Datenaufbereitungs- undDarstellungseinheit auf (nicht dargestellt). Als Übertragungsstrecke kommen alle bekannten Übertragungsstrecken wie Datenleitungen, Modem, Funkstrecke, Satellitenübertragung usw. in Frage. Neben der Datenerfassung und der Korrelationsauswertung steuert der Rechner automatisch nach einem vorgegebenen und gespeicherten Programm Pumpe, Ventil, Leuchtdioden und alle weiteren Einrichtungen und erzeugt den Meßlicht- und den x-Hz-Lichttakt, für die Leuchtdioden wie in Fig. 3a, und Fig. 3b gezeigt.

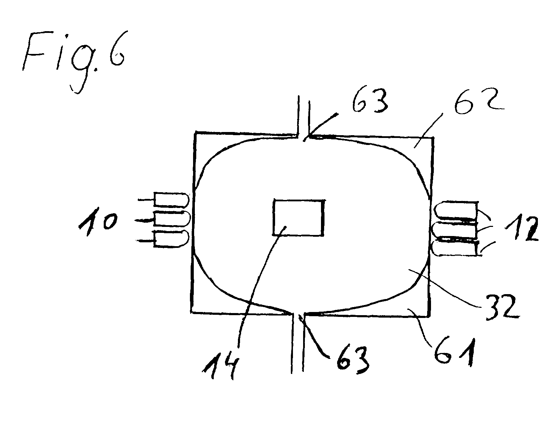

Fig. 6 zeigt eine zu Fig. 2 alternative Skizze einer Meßkammer mit einenm Innenvolumen von etwa 15 Kubikzentimeter. Die dargestellte Meßkammer ist innenseitig reflexionsfrei oder - schwach und weist an ihren oberen und unteren Ecken und Kanten Anrundungen 61 und 62 auf, durch die schwebende und sinkende Teilchen in der Meßprobe automatisch auf einen am Kammerboden vorgesehen Ablaß 63 geleitet werden. Gleichzeitig können Luft- und andere Gasbläschen durch ein Entlüftungsablaß 64 an der Kammerdecke ausgelassen werden.

Darüber hinaus sind wie in Fig 2 bereits gezeigt, mehrere Leuchtdioden gegenüberliegend oder nahezu gegenüberliegend angeordnet. Auf jeder Seite der Meßkammer ist eine rotes, eine blaues, eine grünes, eine gelbes und eine oranges Licht erzeugende Leuchtdiode angeordnet. Durch die sequentielle Ansteuerung der Leuchtdiodenpaare kann aus dem unterschiedlichen Fluoeszenz- und Korrelationsverhalten der Algen bei unterschiedlichen Anregungslicht nicht nur die Algenvitalität und Umweltbelastung der Probe, sondern auch die Algenart zumindestens näherungsweise bestimmt werden. Dazu werden Vergleichsmessungen mit bekannten Algenarten ins Verhältnis mit der aktuellen Messung gesetzt. Dieses erspart kostenintensive und umständliche andere Bestimmungsverfahren und ist sehr vorteilhaft bei der Gesamtbeurteilung des zu untersuchenden Biotops.

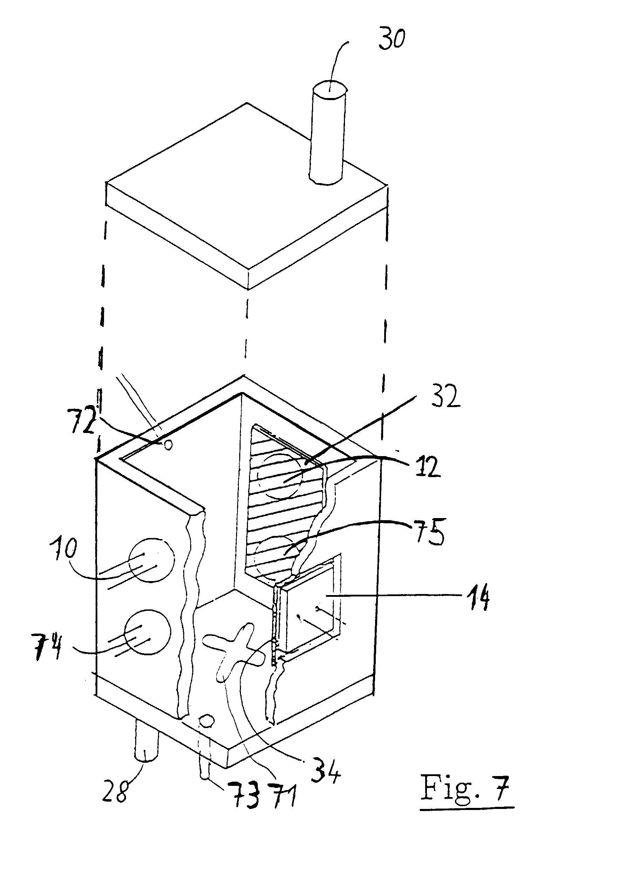

Fig. 7 zeugt eine Skizze einer Meßkammer, die besonders für die Ermittlung der Schadstoffbelastung von Wasser sehr vorteilhaft ist. Die Meßkammer weist einen Rührer 71, sowie einen Injektionseinlaß 72 und einenDruckablaß 73 auf. Insbesondere dann, wenn der Schadstoffgehalt z.B. der mit Nitrat im Wasser bestimmt werden soll läßt sich diese Meßkammer vortrefflich verwenden. Dazu wird zunächst ein Teil Reinwasser mit Algen vermischt und eine erste Vergleichsmessung durchgeführt und die Algenvitalität bestimmt. Anschließend wird statt des Reinwassers mittels einer Injektion ein Teil Nitrat mit Algen zugesetzt und/oder vermischt und eine zweite Vergleichsmessung durchgeführt. Dabei kann aus der Fluoeszenzrückmeldung auf den "Nitrathunger" der Algen geschlossen werden. Schließlich wird ein Teil der zu testende Flüssigkeit, z.B. Abwasser aus einer Kläranlage, in die Meßkammer injiziert, mit den Algen vermischt. Aus der Messung der mit Abwasser versetzten Algen kann durch Vergleich mit den vorhergehenden Messungen sehr sicher und schnell auf die Nitratbelastung des Abwassers geschlossen werden. Andere Schadstoffbestimmungen wie Ammonium, Schwermetalle, Phosphor usw. sind ebenso möglich.

In Fig 7 ist auch eine Atenuationsmeßeinrichtung gezeigt, die aus einer ungetakteten Leuchtdiode 74 und einem gegenüberliegenden Photodetektor 75 besteht, der das auftreffenden Licht mißt und aus der Einfallslichtmenge ein Maß für die Abschwächung des Lichts in der Probe ermittelt. Dieses Maß kann bei der Korrelation bzw. Bestimmung der Algenvitalität berücksichtigt werden..

## Patentansprüche

1. Verfahren zur Messung der Fluoreszenzrückmeldung von Algen zur Algendichte- und Vitalitätsbestimmung mit einer anregenden Beleuchtung, bei dem die Fluoreszenzabstrahlung der untersuchten Pflanzen detektiert wird,
gekennzeichnet durch
- Beleuchtung der Algen gleichzeitig mit zwei verschieden getakteten Lichtsignalen, einem schnell getakteten Meßlicht und einem zweiten langsam getakteten x-Hz-Licht,
- Korrelieren des detektierten Signals mit dem Taktsignal des Meßlichts zum Erhalt eines für die Algendichte maßgeblichen Zwischensignals und
- Korrelieren des Zwischensignals mit dem Taktsignal des x-Hz-Lichts zum Erhalt eines eichbaren, für die Vitalität maßgeblichen Ergebnissignals,
- Integrieren beider Signale.

2. Verfahren nach Anspruch 1, **dadurch gekennzeichnet**, daß unter Verwendung einer Abschwächungsmeßeinrichtung die Abschwächung des Lichtes in den Algen bestimmt wird.

3. Verfahren nach einem der vorhergehenden Ansprüche, **dadurch gekennzeichnet**, daß die Algen nacheinander mit unterschiedlichen Lichtwellenlängen angeregt werden.

4. Verfahren zur Schadstoffbestimmung von Wasser, z.B. Abwasser, nach einem der vorhergehenden Ansprüche, **gekennzeichnet durch** folgende Schritte:
Ein Teil Reinwasser wird mit Algen vermischt und die erste Algenvitalität bestimmt,
ein Teil des zu detektierenden Schadstoffs, z.B. Nitrat, wird mit Algen vermischt und die zweite Algenvitalität bestimmt,
ein Teil des zu untersuchenden Wassers wird mit Algen vermischt und die dritte Algenvitalität bestimmt
die dritte Algenvitalität wird mit den ersten beiden verglichen und der Schadstoffgehalt im zu untersuchenden Wasser bestimmt.

5. Vorrichtung zur Durchführung des Verfahrens nach einem der Ansprüche 1-4 zur Messung der Fluoreszenzrückmeldung von Algen zur Algendichte- und Vitalitätsbestimmung, bei dem eine die Fluoreszenz der Algen anregende Beleuchtung und eine Detektion der Fluoreszenzabstrahlung der untersuchten Pflanzen vorgesehen ist,
dadurch gekennzeichnet, daß
- eine Beleuchtungseinrichtung (10,12) mit zwei gleichzeitig wirksamen, verschieden getakteten Lichtsignalen, einem ersten, schnell getakteten Meßlicht und einem zweiten, langsam getakteten x-Hz-Licht, vorgesehen ist,
- daß ein erster Photodetektor (14) zum Detektieren der beiden Lichtsignale vorgesehen ist,
- daß ein erster, Korrelator (24) zum Korrelieren des detektierten Signals mit dem Taktsignal des Meßlichts zum Erhalt eines für die Algendichte maßgeblichen Zwischensignals und
- ein zweiter Korrelator (24) zum Korrelieren des Zwischensignals mit dem Taktsignal des x-Hz-Lichts zum Erhalt eines eichbaren, für die Vitalität maßgeblichen Ergebnissignals vorgesehen sind,
- je ein Integrator (26) zum Integrieren je eines der beiden Signale vorgesehen ist und daß
- ein an eine Meßkammer (47) angesetzter Temperatursensor zur Messung der Temperatur des Flüssigkeitsvolumens in der Meßkammer vorgesehen ist.

6. Vorrichtung nach Anspruch 5, **dadurch gekennzeichnet**, daß eine Fördereinrichtung vorgesehen und als Schlauchpumpe (43) ausgebildet ist.

7. Vorrichtung nach einem der Ansprüche 5 oder 6, **dadurch gekennzeichnet**, daß an der Meßkammer (16) eine dritte Lichtquelle (74) vorgesehen ist, der ein zweiter Photodetektor (75) zugeordnet ist, der die Dichte und/oder Abschwächung des Lichts der dritten Lichtquelle in den Algen mißt.

8. Vorrichtung nach einem der Ansprüche 5 bis 7, **dadurch gekennzeichnet**, daß die Meßkammer eine Injektionsöffnung (72) und einen dieser zugeordneten Ablaß (73) aufweist.

9. Vorrichtung nach einem der Ansprüche 5 bis 8, **dadurch gekennzeichnet**, daß die Meßkammer eine Mischeinrichtung (71) aufweist.

10. Vorrichtung nach einem der Ansprüche 5 bis 9, **dadurch gekennzeichnet**, daß die Vorrichtung eine oder mehrere der folgenden Einrichtungen aufweist: eine Attenuationsmeßeinrichtung (52), eine Druckmeßeinrichtung (53), eine Leitfähigkeitsmeßeinrichtung (54), eine Sauerstoff- oder andere Gasmeßeinrichtung (55), eine Schaltuhr.

11. Meßstation mit einer Vorrichtung nach einem der Ansprüche 5 bis 10, **dadurch gekennzeichnet**, daß die Meßstation eine Meßdatenerfassungs- und/oder auswerteeinrichtung aufweist, die zentral alle Einrichtungen der Vorrichtung steuert, gemessene Daten empfängt und die gemessenen Daten in einer Speichereinrichtung (58) speichert.

## Claims

1. Method of measuring the fluorescence feedback of algae in order to determine the density and vitality of the algae with an exciting light, the fluorescent radiation of the tested plants being detected, characterised by
- exposing the algae simultaneously to two differently timed light signals, a measurement light timed at a quick rhythm and a second x-Hz light timed at a slow rhythm,
- correlating the detected signal with the timing signal of the measurement light in order to obtain an intermediate signal which is definitive for the density of the algae, and
- correlating the intermediate signal with the timing signal of the x-Hz light in order to obtain a calibratable result signal which is definitive for the vitality,
- integrating both signals.

2. Method as claimed in Claim 1, characterised in that the attenuation of the light in the algae is determined using an attenuation measuring device.

3. Method as claimed in one of the preceding claims, characterised in that the algae are successively excited with different light wavelengths.

4. Method of determining the pollutants in water, e.g. waste water, as claimed in one of the preceding claims, characterised by the following steps:
a proportion of clean water is mixed with algae and the first algae vitality value is determined,
a proportion of the pollutant to be detected, e.g. nitrate, is mixed with algae and the second algae vitality value is determined,
a proportion of the water to be tested is mixed with algae and the third algae vitality value is determined,
the third algae vitality value is compared with the first two and the pollutant content in the water to be tested is determined.

5. Apparatus for carrying out the method as claimed in one of Claims 1 to 4 for measuring the fluorescence feedback of algae in order to determine the density and vitality of the algae, in which a light which excites the fluorescence of the algae is provided and the fluorescent radiation of the tested plants is detected, characterised in that
- a lighting device (10, 12) is provided which has two simultaneously effective and differently timed light signals, a first measurement light timed at a quick rhythm and a second x-Hz light timed at a slow rhythm,
- a first photodetector (14) is provided for detecting the two light signals,
- a first correlator (24) is provided for correlating the detected signal with the timing signal of the measurement light in order to obtain an intermediate signal which is definitive for the density of the algae, and
- a second correlator (24) is provided for correlating the
- a second correlator (24) is provided for correlating the intermediate signal with the timing signal of the x-Hz light in order to obtain a calibratable result signal which is definitive for the vitality,
- an integrator (26) is provided in each case for integrating each of the two signals, and
- a temperature sensor connected to a measurement chamber (47) for measuring the temperature of the volume of fluid in the measurement chamber is provided.

6. Apparatus as claimed in Claim 5, characterised in that a conveying device is provided and is constructed as a hose pump (43).

7. Apparatus as claimed in one of Claims 5 or 6, characterised in that on the measurement chamber (16) there is disposed a third light source (74) with which is associated a second photodetector (75) which measures the density and/or the attenuation of the light of the third light source in the algae.

8. Apparatus as claimed in one of Claims 5 to 7, characterised in that the measurement chamber has an injection opening (72) and an outlet (73) associated therewith.

9. Apparatus as claimed in one of Claims 5 to 8, characterised in that the measurement chamber has a mixing device (71).

10. Apparatus as claimed in one of Claims 5 to 9, characterised in that the apparatus has one or more of the following devices: an attenuation measuring device (52), a pressure measuring device (53), a conductivity measuring device (54), an oxygen or other gas measuring device (55), a switch clock.

11. Measurement station with apparatus as claimed in one of Claims 5 to 10, characterised in that the measurement station has a measurement data gathering and/or evaluating device which centrally controls all devices of the apparatus, receives measured data and stores the measured data in a storage device (58).

12. Measurement station with apparatus as claimed in one of Claims 5 to 10, characterised in that the measurement station has a measurement data gathering and/or evaluating device which centrally controls all devices of the apparatus, receives measured data and stores the measured data in a storage device (58).

## Revendications

1. Procédé de mesure de la fluorescence en retour d'algues afin de déterminer la densité et la vitalité des algues avec un éclairement d'excitation, selon lequel on détecte le rayonnement de fluorescence des plantes analysées,
**caractérisé** par
- éclairement des algues simultanément avec deux signaux lumineux de cadences différentes, une lumière de mesure à cadence rapide et une seconde lumière x-Hz à cadence lente,
- corrélation du signal détecté avec le signal de cadence de la lumière de mesure, afin d'obtenir un signal intermédiaire déterminant de la densité des algues, et
- corrélation du signal intermédiaire avec le signal de cadence de la lumière x-Hz, afin d'obtenir un signal de résultat étalonnable, déterminant de la vitalité,
- intégration des deux signaux.

2. Procédé selon la revendication 1, **caractérisé** en ce qu'en utilisant un dispositif de mesure d'affaiblissement, on détermine l'affaiblissement de la lumière dans les algues.

3. Procédé selon une des revendications précédentes, **caractérisé** en ce que les algues sont excitées successivement avec des longueurs d'ondes lumineuses différentes.

4. Procédé pour la détermination de substances nocives dans de l'eau, par exemple dans des eaux usées, selon une des revendications précédentes, **caractérisé** par les étapes suivantes :
- une partie d'eau pure est mélangée aux algues, et on détermine la première vitalité des algues,
- une partie de la substance nocive à détecter, nitrates par exemple, est mélangée aux algues, et on détermine la deuxième vitalité des algues,
- une partie de l'eau à analyser est mélangée aux algues, et on détermine la troisième vitalité des algues,
- la troisième vitalité des algues est comparée aux deux premières, et on détermine la teneur en substances nocives de l'eau à analyser.

5. Dispositif pour la mise en oeuvre du procédé selon une des revendications 1 à 4 pour la mesure de la fluorescence en retour d'algues afin de déterminer la densité et la vitalité des algues, selon lequel il est prévu un éclairement excitant la fluorescence des algues et une détection du rayonnement de fluorescence des plantes analysées, **caractérisé**
- en ce qu'est prévu un dispositif d'éclairement (10, 12) avec deux signaux lumineux simultanément actifs, de cadences différentes, une première lumière de mesure, à cadence rapide, et une seconde lumière x-Hz, à cadence lente,
- en ce qu'est prévu un premier photodétecteur (14) pour la détection des deux signaux lumineux,
- en ce que sont prévus un premier corrélateur (24) pour la corrélation du signal détecté avec le signal de cadence de la lumière de mesure, afin d'obtenir un signal intermédiaire déterminant de la densité des algues,
- et un second corrélateur (24) pour la corrélation du signal intermédiaire avec le signal de cadence de la lumière x-Hz, afin d'obtenir un signal de résultat étalonnable, déterminant de la vitalité,
- en ce qu'un intégrateur respectif (26) est prévu pour l'intégration de chacun des deux signaux,
- et en ce qu'est prévu un capteur de température, appliqué à une chambre de mesure (47), pour la mesure de la température du volume de liquide dans la chambre de mesure.

6. Dispositif selon la revendication 5, **caractérisé** en ce qu'est prévu un dispositif de refoulement, qui est réalisé sous forme de pompe tubulaire (43).

7. Dispositif selon la revendication 5 ou 6, **caractérisé** en ce qu'une troisième source lumineuse (74) est prévue sur la chambre de mesure (16), source à laquelle est associée un deuxième photodétecteur (75) qui mesure la densité et/ou l'affaiblissement de la lumière de la troisième source lumineuse dans les algues.

8. Dispositif selon une des revendications 5 à 7, **caractérisé** en ce que la chambre de mesure présente une ouverture d'injection (72), et une évacuation (73) associée à cette ouverture.

9. Dispositif selon une des revendications 5 à 8, **caractérisé** en ce que la chambre de mesure présente un dispositif mélangeur (71).

10. Dispositif selon une des revendications 5 à 9, **caractérisé** en ce que le dispositif présente un ou plusieurs des équipements suivants: un équipement de mesure d'atténuation (52), un équipement de mesure de pression (53), un équipement de mesure de conductibilité (54), un équipement de mesure d'oxygène ou autres gaz (55), un interrupteur horaire.

11. Station de mesure avec un dispositif selon une des revendications 5 à 10, **caractérisée** en ce que la station de mesure présente un équipement d'enregistrement et/ou d'interprétation des données de mesure, qui commande centralement tous les équipements du dispositif, reçoit les données mesurées et mémorise les données mesurées dans un équipement de mémorisation (58).
